# EUROPÄISCHE PATENTSCHRIFT

(11) **EP 1 456 162 B1**
(45) Veröffentlichungstag und Bekanntmachung des Hinweises auf die Patenterteilung: **17.10.2007**
(21) Anmeldenummer: 02792810.0
(22) Anmeldetag: 27.11.2002
(51) Int. Cl.: C07C 45/50, C07C 47/02

(54) **VERFAHREN ZUR HERSTELLUNG VON ALDEHYDEN**
METHOD FOR THE PRODUCTION OF ALDEHYDES
PROCEDE POUR PREPARER DES ALDEHYDES

(30) Priorität: 08.12.2001 DE 10160368
(43) Veröffentlichungstag der Anmeldung: 15.09.2004
(73) Patentinhaber: OXEA GmbH, 46147 Oberhausen (DE)
(72) Erfinder: KRUMREY, Thomas, 40882 Ratingen (DE); SCHALAPSKI, Kurt, 46147 Oberhausen (DE); RAPIER, Robert, Ponca City, OK 74601 (US); STEINBACH, John, League City, TX 77573 (US); SHAH, Bak, Bay City, TX 77414 (US); HEWLETT, Mark, Bay City, TX 77414 (US)
(74) Vertreter: Szameitat, Jürgen
(86) Internationale Anmeldenummer: PCT/EP2002/013340
(87) Internationale Veröffentlichungsnummer: WO 2003/050068

(56) Entgegenhaltungen:
- DE-A- 4 419 898
- US-A- 5 648 553

## Beschreibung

Die Erfindung betrifft ein Verfahren zur Hydroformylierung olefinisch ungesättigter Verbindungen, wobei der den Hydroformylierungsreaktor verlassende Katalysator und Aldehyd haltige homogene, flüssige Austrag in einer nachgeschalteten Extraktionskolonne im Gegenstrom mit Synthesegas bei erhöhter Temperatur behandelt wird.

Es ist bekannt, Verbindungen, die olefinische Doppelbindungen enthalten, mit Kohlenmonoxid und Wasserstoff zu Aldehyden umzusetzen (Oxosynthese). Der Prozeß ist nicht auf den Einsatz olefinischer Kohlenwasserstoffe begrenzt, sondern erstreckt sich auch auf Ausgangsstoffe, die außer der Doppelbindung noch funktionelle Gruppen aufweisen, vorwiegend solche, die unter den Reaktionsbedingungen unverändert bleiben.

Die klassische Oxosynthese arbeitet mit Kobalt als Katalysator. Seine Wirksamkeit beruht auf der Bildung von Kobaltcarbonylverbindungen unter der Einwirkung von Wasserstoff und Kohlenmonoxid bei Drücken oberhalb 20 MPa und Temperaturen von etwa 120°C und mehr auf metallisches Kobalt oder Kobaltverbindungen.

Im Laufe der Weiterentwicklung der Oxosynthese wurde Kobalt zunehmend durch Rhodium als Katalysatormetall ersetzt. Rhodium wird als Komplexverbindung eingesetzt, das neben Kohlenmonoxid vorzugsweise organische Phosphine als Liganden enthält. Rhodium als Metall erlaubt es, bei niedrigen Drücken zu arbeiten, überdies erzielt man höhere Ausbeuten und bevorzugt werden die für die Weiterverarbeitung wertvolleren unverzweigten Produkte gebildet, wenn man von geradkettigen endständigen Olefinen ausgeht.

Ein derartiges Verfahren ist aus US-A-3,527,809 unter der Bezeichnung Niederdruck-Rhodium-Verfahren bekannt. Eine Verbesserung dieses Prozesses offenbart die US-A-4,148,830. Nach dieser Verfahrensweise kann die Katalysatorlebensdauer und die Ausbeute an linearen Aldehyden erhöht werden, wenn man als Lösungsmittel für den Katalysator und das im Überschuß vorhandene organische Phosphin die hochsiedenden Kondensationsprodukte der gebildeten Aldehyde verwendet. Dabei läßt sich die Abscheidung unlöslicher Rhodiumverbindungen vermeiden und der gelöste Katalysator läßt sich über viele Katalysezyklen wiederverwenden, ohne daß man ein Nachlassen in der Aktivität beobachtet. Nach dem aus US-A-4,148,830 bekannten Verfahren wird der flüssige Austrag aus dem Hydroformylierungsreaktor zunächst gekühlt und unter Entspannung durch einen Gas/Flüssigabscheider geleitet, wodurch im Rohaldehyd gelöster Wasserstoff, gelöstes Kohlenmonoxid und nicht reagierte olefinische Verbindungen verdampfen. Anschließend wird der Katalysator haltige Rohaldehyd durch einen Verdampfer geleitet, wobei der Rohaldehyd als Kopffraktion gewonnen wird und ein Rhodium haltiger Rückstand anfällt, der freie Phosphinliganden, Aldehydreste und höhersiedende Aldehydkondensationsprodukte enthält und der in den Hydroformylierungsprozeß zurückgefahren werden kann. Das bei der Katalysatorabtrennung erhaltene Aldehyd haltige Kopfprodukt wird in einer nachgeschalteten Destillation weiter gereinigt und in die n- und iso-Verbindung aufgetrennt. Das aus US-A-4,148,830 bekannte Verfahren wird auch als "Hydroformylierungsverfahren unter Flüssigkeitsrückführung" bezeichnet.

Ein weiteres in Gegenwart von Rhodium und organischen Phosphinen durchgeführtes Hydroformylierungsverfahren ist aus US-A-4,247,486 bekannt. Bei dieser Verfahrensvariante wird dem Reaktor ein gasförmiger Produktstrom entnommen, der die olefinisch ungesättigte Verbindung, Wasserstoff, verdampften Rohaldehyd und seine Kondensationsprodukte enthält. Dieser gasförmige Produktstrom wird kondensiert und einem Trenngefäß zugeleitet, in dem sich der Rohaldehyd von den flüchtigen Bestandteilen abscheidet. Die flüchtigen Bestandteile, bei denen es sich im wesentlichen um Wasserstoff, Kohlenmonoxid, die olefinisch ungesättigte Verbindung und ihr Hydrierprodukt handelt, werden nach Abtrennung der Hydrierprodukte, beispielsweise der Alkane, wieder in den Hydroformylierungsreaktor zurückgeführt. Die Menge des pro Zeiteinheit zurückgeführten Gases ist so bemessen, dass das Flüssigkeitsvolumen in dem Hydroformylierungsreaktor konstant bleibt. Ferner entspricht die mit dem gasförmigen Reaktoraustrag entfernte Menge an Aldehydkondensationsprodukten der während der Hydroformylierungsreaktion gebildeten Menge. Das aus US-A-4,247,486 bekannte Verfahren wird auch als "Hydroformylierungsverfahren unter Gasrückführung" bezeichnet.

DE-A1-44 19 898 behandelt ein Verfahren zur Herstellung von Aldehyden, bei dem der flüssige Reaktoraustrag in einer Extraktionskolonne mit Synthesegas im Gegenstrom in Kontakt gebracht wird, um nicht umgesetztes Olefin ohne wesentliche Desaktivierung des Katalysators abzutrennen und wieder in die Hydroformylierungszone zurückzuführen.

Eine derartige Stabilisierung des Katalysators kann durch Einstellen der Verweilzeiten in der Extraktionskolonne, durch Einstellen der Bodentemperatur oder durch zusätzliche Olefinzugabe über den Boden der Extraktionskolonne erfolgen, wobei sich das Molverhältnis von Olefin zu Rhodium vorzugsweise in einem Bereich von 1,0 bis 50 bewegen soll.

Aus US-A-5 648 553 ist bekannt, den flüssigen Reaktoraustrag vor dem Eintritt in die mit Synthesegas betriebene Extraktionskolonne auf eine Temperatur von 130 bis 190°C aufzuheizen, um eine wirksamere Olefinabtrennung in der Extraktionskolonne zu erzielen.

Es sind ebenfalls Hydroformylierungsverfahren bekannt, in denen das Abgas einer ersten Hydroformylierungsstufe in einer zweiten Hydroformylierungsstufe weiter umgesetzt wird. Ein solches Verfahren wird in der EP-A1-0 188 246 beschrieben. Dabei wird in der ersten Stufe unter Rückführung von Flüssigkeit, wie aus US-A-4,148,830 bekannt, gearbeitet und das anfallende Abgas einer zweiten entkoppelten Stufe, d.h. von der ersten Stufe separat betriebenen sekundären Stufe, zugeleitet, in dem unter Rückführung von Flüssigkeit oder Gas das Abgas der ersten Hydroformylierungsstufe zusammen mit zugesetztem Kohlenmonoxid und Wasserstoff zur Reaktion gebracht wird.

EP 0188 246 weist daraufhin, dass bei der nach der Flüssigkeitsrückführung durchgeführten ersten Hydroformylierungsstufe in der in dem nachgeschalteten Gas/Flüssigabscheider angefallenen Lösung olefinisch ungesättigte Verbindungen und Synthesegas gelöst enthalten sind. Diese und weitere flüchtige Bestandteile können zusammen mit dem Rohaldehyd in einem Verdampfer von der Katalysatorlösung abdestilliert werden und kondensieren zum Teil in dem Rohaldehyd. Diese Anteile an flüchtigen Verbindungen werden bei der nachfolgenden Reinigung des Rohaldehyds in das n- und iso-Isomer zurückgewonnen und in den Hydroformylierungsprozeß zurückgefahren.

Die bei der Abtrennung des Rohaldehyds von der Katalysatorlösung nicht im Rohaldehyd kondensierten flüchtigen Anteile werden abgetrennt, komprimiert und direkt in den Hydroformylierungsprozeß wieder zurückgefahren.

US 5,105,018 betrifft ebenfalls ein zweistufiges Hydroformylierungsverfahren, in dem in der ersten Reaktionsstufe der gasförmige oder flüssig/gasförmige Reaktoraustrag zunächst gekühlt wird und anschließend in einen Gas/Flüssigabscheider geleitet wird, aus dem das Abgas in die erste Hydroformylierungsstufe zurückgeführt wird, während die gekühlte Lösung, die Ausgangsolefin gelöst enthält, in einer Extraktionskolonne im Gegenstrom mit Synthesegas behandelt wird, wodurch gelöstes Olefin ausgetrieben und zusammen mit Synthesegas in die erste Hydroformylierungsstufe zurückgeführt wird. Das Abgas der ersten Hydroformylierungsstufe wird einer zweiten Hydroformylierungsstufe zugeleitet, in der der gas/flüssige Reaktoraustrag ebenfalls zunächst gekühlt wird und auf einen Gas/Flüssigabscheider gegeben wird. Die in dem Gas/Flüssigabscheider der zweiten Hydroformylierungsstufe anfallende Rohaldehyd und Katalysatorlösung enthaltende Flüssigkeit wird mit der nach der Synthesegasextraktion in der ersten Stufe anfallenden Lösung vereinigt und auf einen Verdampfer gegeben, in dem die Abtrennung von Rohaldehyd und Katalysatorlösung erfolgt. Die Katalysatorlösung wird in die erste und zweite Hydroformylierungsstufe zurückgefahren.

Aus dem Stand der Technik ist bekannt, den flüssigen, auf Reaktionstemperatur erhitzten Reaktoraustrag, auf 60 oder 80°C zu kühlen und auf einen Gas/Flüssigabscheider zu geben. Der dabei anfallende flüssige Stoffstrom enthält eine erhebliche Menge an nicht reagierter olefinischer Ausgangsverbindung, die nach dem Stand der Technik über eine mit Synthesegas betriebene Extraktionskolonne aus der Flüssigkeit entfernt und in den Hydroformylierungsreaktor zurückgeleitet wird.

Eine hohe Restmenge an olefinisch ungesättigter Verbindung in dem nach dem Gas/Flüssigabscheider anfallenden Reaktionsprodukt wirkt sich jedoch auf die nachfolgenden Aufarbeitungs- und Trennschritte nachteilig aus. Ein hoher Restgehalt an olefinisch ungesättigter Verbindung erfordert in der Extraktionskolonne eine hohe Synthesegaszufuhr bei hohem Druck, um die gelöste olefinisch ungesättigte Verbindung möglichst vollständig auszutreiben. Eine solche möglichst vollständige Entfernung der flüchtigen olefinischen Verbindungen ist ebenfalls für den Betrieb der nachgeschalteten destillativen Abtrennung des Rohaldehyds aus der Katalysatorlösung sowie für die destillative Reinigung der n- und iso-Aldehyde zweckmäßig, da hohe Restgehalte an leicht flüchtigen olefinischen Verbindungen die Temperatur- und Druckverhältnisse in den nachgeschalteten Destillationen nachteilig beeinflussen. Die an diesen Stellen des Prozesses zurückgewonnene olefinische Verbindung muß zunächst unter hohem Energieaufwand komprimiert werden, um in den Hydroformylierungsreaktor zurückgefahren werden zu können. Im allgemeinen enthält die in dem Gas/Flüssigabscheider anfallende flüssige, homogenen Lösung bis zu 10 Gew.-%, häufig bis zu 8 Gew.-% olefinisch ungesättigte Verbindung, bezogen auf die gesamte Lösung. Wünschenswert ist es, diese Restmenge an olefinisch ungesättigter Verbindung möglichst einfach in Aldehydwertprodukt zu überführen, um den Restgehalt an flüchtigen olefinischen Verbindungen in den flüssigen Stoffströmen, die der destillativen Aufarbeitung zugeführt werden, möglichst gering zu halten, und um damit die zuvor genannten Nachteile zu vermeiden.

Es bestand daher die Aufgabe, ein Hydroformylierungsverfahren für olefinisch ungesättigte Verbindungen bereitzustellen, bei dem die in dem flüssigen, homogenen Reaktoraustrag enthaltene Restmenge an olefinisch ungesättigter Verbindung möglichst einfach zu Aldehyden umgesetzt wird und damit der Restgehalt an olefinisch ungesättigter Verbindung in den der destillativen Aufarbeitung zugeführten flüssigen Stoffströmen vermindert wird.

Diese Aufgabe wird gelöst durch ein Verfahren zur Umsetzung olefinisch ungesättigter Verbindungen mit Synthesegas in Gegenwart einer Rhodium und organische Phosphor(III)-Verbindungen enthaltenden Katalysatorlösung in einem Hydroformylierungsreaktor, bei dem der homogene, flüssige Rohaldehyd und Katalysator enthaltende Reaktoraustrag auf einen Gas/Flüssigab scheider gegeben wird und der erhaltene homogene, flüssige Stoffstrom in einer mit Synthesegas im Gegenstrom betriebenen Extraktionskolonne behandelt wird und der flüssige Austrag aus der Extraktionskolonne in einen Verdampfer geleitet wird, in dem Rohaldehyd und Katalysatorlösung voneinander getrennt werden. Es ist dadurch gekennzeichnet, dass dem nach dem Gas/Flüssigabscheider anfallenden homogenen, flüssigen Stoffstrom vor dem Eintritt in die Extraktionskolonne Synthesegas zugesetzt wird und der homogene, flüssige Stoffstrom bei einer Temperatur von 110 bis 150°C auf den oberen Teil der Extraktionskolonne gegeben wird.

Das erfindungsgemäße Hydroformylierungsverfahren wird unter Flüssigkeitsrückführung durchgeführt. Der flüssige Reaktoraustrag wird zunächst ohne Kühlung durch einen Gas/Flüssigabscheider geleitet, in dem aus dem flüssigen Stoffstrom die gasförmigen Anteile verdampfen und als Abgas aus dem Reaktionssystem entfernt werden. Im Gas/Flüssigabscheider stellt sich die Temperatur ein, die der homogene, flüssige Reaktoraustrag aufweist, sie liegt im allgemeinen im Bereich von 110 bis 150°C. Die Druckbedingungen entsprechen denen im Hydroformylierungsreaktor.

Der den Gas/Flüssigabscheider verlassende flüssige Stoffstrom aus Rohaldehyd und Katalysatorlösung enthält ferner gelöst Synthesegas und olefinisch ungesättigte Verbindung. Im allgemeinen beträgt der Gehalt an olefinisch ungesättigter Verbindung bis zu 10 Gew.-%, in den meisten Fällen 4-8 Gew.-%, bezogen auf die homogene Reaktionsmischung.

Im Gegensatz zu der Lehre aus dem Stand der Technik wird der flüssige Reaktoraustrag ohne Kühlung auf den Gas/Flüssigabscheider gegeben. Es ist wesentlich, dass die nach dem Flüssigkeitsabscheider anfallende homogene Lösung bei einer Temperatur von 110 bis 150°C und insbesondere von 115 bis 145°C auf den oberen Teil der Extraktionskolonne gegeben wird.

Die Aufgabe erfolgt im allgemeinen auf den oberen Teil der Extraktionskolonne, vorzugsweise auf dem 1. Boden, gerechnet vom Kolonnenkopf. Synthesegas strömt dem flüssigen Gemisch aus Rohaldehyd und Katalysatorlösung vom Boden her entgegen, und wird am Kopf der Extraktionskolonne entnommen und dem Hydroformylierungsreaktor wieder zugeführt. Die Extraktionskolonne wird im allgemeinen bei einer Temperatur betrieben, die etwas unterhalb der Reaktortemperatur liegt. Sie beträgt im allgemeinen 80 bis 120°C, vorzugsweise 90 bis 110°C. Da Synthesegas durch die Extraktionskolonne in den Hydroformylierungsreaktor hineingedrückt werden muß, ist in der Extraktionskolonne im Vergleich zu den Druckbedingungen im Hydroformylierungsreaktor ein erhöhter Synthesegasdruck einzustellen, der im allgemeinen den Druck im Hydroformylierungsreaktor um 0,1 bis 1,0 MPa übersteigt.

Durch die erfindungsgemäße Zugabe der nach dem Gas/Flüssigabscheider anfallenden homogenen Lösung auf die Extraktionskolonne bei erhöhter Temperatur erfolgt in Anwesenheit der Katalysatorlösung ein weiterer Restumsatz der gelösten olefinisch ungesättigten Verbindung mit dem im Gegenstrom sowie zusätzlich zugeführten Synthesegas zu Aldehyden. Bei der erfindungsgenmäßen Verfahrensdurchführung arbeitet die Extraktionskolonne somit einmal als Destillationskolonne zur Abtrennung von olefinisch ungesättigten Verbindungen über den Kopf der Extraktionskolonne und als Reaktionskolonne, wobei die olefinisch ungesättigten Verbindungen zu weniger flüchtigen Aldehyden abreagieren, die man mit dem flüssigen Austrag über den Boden der Extraktionskolonne dem Rohaldehydverdampfer zuführt.

Da in der Extraktionskolonne neben der destillativen Abtrennung der olefinisch ungesättigten Verbindung ebenfalls die Umsetzung zu Aldehyden zu erfolgen hat, ist sicherzustellen, dass in der Extraktionskolonne eine ausreichend hohe Temperatur aufrecht erhalten wird, um die Hydroformylierungsreaktion aufrecht zuerhalten. Dies wird im allgemeinen schon dadurch erreicht, dass durch die Reaktion in der Extraktionskolonne Reaktionswärme freigesetzt wird. Durch die Aufgabe der homogenen Lösung auf den oberen Teil der Extraktionskolonne bei einer Temperatur im Bereich von 110 bis 150°C läßt sich in der Extraktionskolonne über eine Zone von etwa 20 %, bezogen auf die Länge der Extraktionskolonne und gerechnet vom Kolonnenkopf, die zur Hydroformylierungsreaktion erforderliche Reaktionstemperatur aufrecht erhalten.

Da die Verdampfung der olefinisch ungesättigten Verbindung in der Extraktionskolonne eine Kühlung bewirkt, wird die auf die Extraktionskolonne zu gebende flüssige homogene Lösung zuvor in der Zuführungsleitung mit Synthesegas versetzt und anschließend auf die Extraktionskolonne gegeben. Da der flüssige, homogene Reaktoraustrag nicht gekühlt wird und sich weiterhin auf einer Temperatur befindet, die nur wenig unterhalb der eigentlichen Hydroformylierungstemperatur liegt, kommt es schon in der Zuführungsleitung zu der Extraktionskolonne aufgrund der Anwesenheit des gelösten Katalysators zu einer weiteren Reaktion zwischen der gelösten olefinisch ungesättigten Verbindung und dem zugesetzten Synthesegas unter Freisetzung von Reaktionswärme. Da die Verweilzeit in der Zuführungsleitung zu der Extraktionskolonne nur gering ist und die durch die freigesetzte Reaktionswärme zusätzlich erhitzte Lösung nahezu unverzüglich auf die Extraktionskolonne gegeben wird, ist eine Katalysatorschädigung an dieser Stelle des Prozesses nicht zu befürchten. Durch die Aufgabe der durch die Reaktionswärme zusätzlich erhitzten homogenen, flüssigen Lösung auf die Extraktionskolonne läßt sich auf einfache Weise das Temperaturprofil in der Extraktionskolonne noch weiter erhöhen. Dadurch verläßt auch der homogene flüssige Stoffstrom die Extraktionskolonne mit einer höheren Temperatur, so daß in dem nachgeschaltenen Verdampfer zur Rohaldehyd- und Katalysatorabtrennung ein geringerer Energieaufand benötigt wird.

Durch die erfindungsgemäße Maßnahme, in der Extraktionskolonne die Entfernung der olefinisch ungesättigten Verbindung nicht nur durch einen physikalischen Destillationsprozeß zu erzielen, sondern eine weitere Umsetzung von olefinisch ungesättigter Verbindung mit Synthesegas zu Aldehyd durchzuführen, lassen sich vielfältige Vorteile erzielen. Da der auf die Extraktionskolonne zu gebenden flüssigen Stoffstrom nicht gekühlt wird sondern bei erhöhter Temperatur aufgegeben wird und die freigesetzte Reaktionswärme zusätzliche Wärme liefert, kann die Extraktionskolonne bei erhöhter Temperatur betrieben werden, ohne dass eine zusätzliche externe Energiequelle benötigt wird. Daher läßt sich die olefinisch ungesättigte Verbindung in hohem Maße in der bei erhöhter Temperatur betriebenen Extraktionskolonne entfernen, so daß der Restgehalt an olefinisch ungesättigter Verbindung in dem die Extraktionskolonne verlassenden flüssigen Stoffstrom reduziert wird mit dem Vorteil, dass über den nachgeschalteten Rohaldehydverdampfer weniger olefinisch ungesättigte Verbindung in die nachfolgenden Prozeßstufen gelangt. Im allgemeinen liegt der Restgehalt an olefinisch ungesättigter Verbindung in der auf den Rohaldehydverdampfer gegebenen homogenen Lösung bei weniger als 0,7 Gew.-%, vorzugsweise bei weniger als 0,5 Gew.-%, jeweils bezogen auf die homogene Lösung. Da in der Extraktionskolonne eine weitere Umsetzung der olefinisch ungesättigten Verbindung zu Aldehyd erfolgt, führt die erfindungsgemäße Maßnahme zu einer Erhöhung des Reaktorvolumens und damit in sehr einfacher Weise zu einer Kapazitätserhöhung. Schließlich wird Reaktionswärme auf den homogenen flüssigen Austrag aus der Extraktionskolonne übertragen, der dann mit einer erhöhten Temperatur auf den Rohaldehydverdampfer gegeben werden kann. Dadurch läßt sich der externe Energiebedarf am Rohaldehydverdampfer reduzieren.

Besonders geeignet für die Durchführung des erfindungsgemäßen Verfahrens sind solche Extraktionskolonnen, die mit Füllkörpern wie Ring- oder Sattelkörpern oder Stahlwendeln gefüllt sind. Im allgemeinen besitzt die Extraktionskolonne 10 bis 40, vorzugsweise 15 bis 25 Trennstufen.

Der über den Boden die Extraktionskolonne verlassende Katalysator und Rohaldehyd enthaltende flüssige Stoffstrom wird auf einen Verdampfer gegeben, in dem über Kopf Rohaldehyd abgezogen wird, während die Katalysatorlösung als Sumpf anfällt und in den Hydroformylierungsreaktor zurückgeschleust wird. Da der auf den Verdampfer gegebene homogene, flüssige Stoffstrom durch die zuvor beschriebenen erfinderischen Maßnahmen nur noch geringe Restgehalte an olefinisch ungesättigter Verbindung enthält, können die Druck- und Temperaturbedingungen im Verdampfer vorteilhafterweise in einem Bereich im allgemeinen von 115 bis 160°C und vom Vakuum bis Normaldruck eingestellt werden. Zweckmäßiger Weise sind die Bedingungen so zu wählen, dass über Kopf möglichst wenig organische Phosphor(III)-Verbindungen und Rhodium ausgetragen werden und die thermische Zersetzung von Rhodiumkomplexverbindungen im Verdampfer möglichst vermieden wird.

Der abgetrennte Rohaldehyd wird in an sich bekannter Weise destillativ aufgearbeitet und in n- und iso-Aldehyd getrennt.

Man führt die Hydroformylierung in einem homogenen Reaktionssystem durch. Der Begriff homogenes Reaktionssytem steht für eine im wesentlichen aus Lösungsmittel, Katalysator, olefinisch ungesättigter Verbindung und Reaktionsprodukt zusammengesetzte homogene Lösung. Als besonders wirksame Lösungsmittel haben sich die höher siedenden Kondensationsverbindungen der herzustellenden Aldehyde, insbesondere die Trimeren und Tetrameren der herzustellenden Aldehyde, erwiesen, die als Nebenprodukte bei der Hydroformylierung anfallen, sowie ihre Mischungen mit den herzustellenden Aldehyden, so daß ein weiterer Lösungsmittelzusatz nicht unbedingt erforderlich ist. In einigen Fällen kann sich jedoch ein Lösungsmittelzusatz als zweckmäßig erweisen. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe, wie Benzol und Toluol oder die Xylole. Andere gebräuchliche Lösungsmittel sind Paraffinöl, Cyclohexan, n-Hexan, n-Heptan oder n-Octan, Ether, wie Tetrahydrofuran, Ketone, Texanol® der Firma Eastman oder Alkylendiole wie Ethylenglykol, Diethylenglykol, Triethylenglykol, 1,2-Propandiol, 1,4- Butandiol oder Alkylenglykole mit einer mittleren Molmasse im Bereich von 200 bis 1000 g/mol. Der Anteil des Lösungsmittels im Reaktionsmedium kann über einen weiten Bereich variiert werden und beträgt üblicherweise zwischen 20 und 90 Gew.-%, vorzugsweise 50 bis 80 Gew.-%, bezogen auf das Reaktionsgemisch.

Als Katalysatoren werden Rhodium-Komplexverbindungen verwendet, die organische Phosphor(III)-Verbindungen als Liganden enthalten. Derartige Komplexverbindungen und ihre Herstellung sind bekannt (z.B. aus US-A-3 527 809, US-A-4 148 830, US-A-4 247 486, US-A-4 283 562). Sie können als einheitliche Komplexverbindungen oder auch als Gemisch unterschiedlicher Komplexverbindungen eingesetzt werden. Die Rhodium-Konzentration im Reaktionsmedium erstreckt sich über einen Bereich von etwa 1 bis etwa 1000 Gew.-ppm und beträgt vorzugsweise 10 bis 800 Gew.-ppm. Inbesondere wendet man Rhodium in Konzentrationen von 100 bis 700 Gew.-ppm, jeweils bezogen auf die homogene Reaktionslösung an. Als Katalysator kann die stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigen Phosphorliganden durchzuführen, der mit Rhodium keine Komplexverbindung mehr eingeht. Der freie Phosphorligand kann der gleiche sein, wie in der Rhodium-Komplexverbindung, es können aber auch von diesem verschiedene Liganden eingesetzt werden. Der freie Ligand kann eine einheitliche Verbindung sein oder aus einem Gemisch verschiedener organischer Phosphor(III)-Verbindungen bestehen. Beispiele für Rhodium-Phosphor-Komplexverbindungen, die als Katalysatoren Anwendung finden können, sind in US-A-3 527 809 beschrieben. Zu den bevorzugten Liganden in den Rhodium-Komplexkatalysatoren zählen z. B. Triarylphosphine wie Triphenylphosphin, Trialkylphosphine wie Tri(n-octyl)phosphin, Trilaurylphosphin, Tri(cyclohexyl)phosphin, Alkylphenylphosphine, Cycloalkylphenylphosphine und Diphosphine, insbesondere solche Diphosphine mit einem aromatischen Rest, wie zum Beispiel 2,2'-Bis(diphenylphosphino)-biphenyl, 2,2'-Bis(diphenylphosphino)-binaphthyl, 2,2'-Bis(diphenylphosphinomethyl)-biphenyl oder 2,2'-Bis(diphenylphosphinomethyl)-binaphthyl.

Wegen seiner leichten Zugänglichkeit wird Triphenylphosphin besonders häufig angewandt. Neben den organischen Phosphinen kann man auch organische Phosphite und Diphosphite als organische Phosphor(III)-Verbindungen einsetzen.

Die Konzentration an organischen Phosphor(III)-Verbindungen beträgt im allgemeinen 15 bis 60 Gew.-%, bezogen auf die homogene Reaktionslösung. Vorzugsweise arbeitet man mit einer Konzentration an organischen Phosphor(III)-Verbindungen im Bereich von 25 bis 50 Gew.-%, insbesondere zwischen 30 bis 40 Gew.-%, jeweils bezogen auf die homogene Reaktionslösung. Überraschenderweise beobachtet man mit zunehmender Konzentration an organischen Phosphor(III)-Verbindungen eine Verschiebung des n/i-Aldehydverhältnisses in Richtung des n-Aldehyds.

Die Hydroformylierungsbedingungen können innerhalb weiter Grenzen varrieren und den individuellen Gegebenheiten angepaßt werden. Sie hängen u.a. vom Einsatzmaterial oder vom ausgewählten Katalysatorsystem ab. Üblicherweise führt man die Hydroformylierung der Einsatzstoffe bei Temperaturen von 90 bis 150°C durch. Bevorzugt hält man Temperaturen von 110 bis 150 °C und insbesondere von 120 bis 140°C ein. Der Gesamtdruck erstreckt sich über einen Bereich von 0,1 bis 10 MPa, vorzugsweise 1 bis 5 MPa und insbesondere 2 bis 2,5 MPa.

Im allgemeinen bildet man den Katalysator aus den Komponenten Rhodium oder Rhodiumverbindung und organischer Phosphor(III)-Verbindung in Gegenwart von Sythesegas in einer Präformierungsstufe. Als besonders geeignete Lösungsmittel, in denen die Präformierung durchgeführt wird, haben sich die hochsiedenden Kondensationsprodukte der Aldehyde erwiesen. Geeignete Lösungsmittel sind ebenfalls Alkylenglykole wie Ethylenglykol oder 1,2-Propandiol. Die Bedingungen des Präformierungsschrittes entsprechen im allgemeinen den Bedingungen in der Hydroformylierungsstufe. Die Präformierungsbedingungen können beim Anfahren des Hydroformylierungsprozesses eingestellt werden, so dass die Zugabe der olefinisch ungesättigten Verbindung erst dann erfolgt, wenn sich der aktive Rhodiumkatalysator gebildet hat. Wird Rhodium während des laufenden Prozesses zugesetzt, so ist in einem separaten Präformierungsschritt zunächst eine Lösung des aktiven Rhodium-Phosphor(III)-Komplexes herzustellen, die anschließend zu dem Prozeß ergänzt wird. Dabei verwendet man vorzugsweise im Präformierungsschritt auch das Lösungsmittel, das in der Hydroformylierungsstufe bereits eingesetzt wird.

Die Hydroformylierungsreaktion kann in den bekannten Reaktorausführungen, wie in einem Rührreaktor, Rohrreaktor, Mehrkammerreaktor oder Schlaufenreaktor durchgeführt werden.

Die Zusammensetzung des zugeführten Synthesegases kann über einen breiten Bereich variiert werden. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet. Es kann sich jedoch auch als zweckmäßig erweisen, ein Wasserstoff reiches Synthesegas einzusetzen. Ein hoher Wasserstoffanteil wirkt stabilisierend auf das Katalysatorsystem.

Auch die Zugabestelle für Synthesegas in den Hydroformylierungsreaktor kann varriert werden, wobei Synthesegas an einer oder an mehreren Einspeisestellen dem Hydroformylierungsreaktor zugeführt wird. Neben der gebräuchlichen Einspeisung von Synthesegas über den unteren Teil des Reaktors, vorzugsweise über den Reaktorboden, kann in einer weiteren Verfahrensvariante Synthesegas auf den mittleren Teil des Hydroformylierungsreaktors gegeben werden. Dabei kann man entweder das gesamte Synthesegas über den unteren Teil oder über den mittleren Teil des Hydroformylierungsreaktors geben oder man speist einen Teil des Synthesegases über den unteren Teil und den anderen Synthesegasteilstrom über den Mittelteil des Hydroformylierungsreaktors ein. Bei der Synthesegaseinspeisung kann man zunächst das in den Prozeß eingeführte frische Synthesegas vollständig auf die Extraktionskolonne geben und das vom oberen Teil der Extraktionskolonne abgeführte und mit olefinisch ungesättigter Verbindung beladene Synthesegas dem Hydroformylierungsreaktor zuführen. Es ist aber auch möglich, den Strom an frischem Synthesegas zunächst zu teilen und einen Teilstrom, der zunächst über die Extraktionskolonne geführt wird, in den Reaktor einzuspeisen, während der andere Teilstrom an frischem Synthesegas direkt in den Hydroformylierungsreaktor geführt wird. Das Verhältnis der Teilströme kann über einen weiten Bereich variiert werden. Im allgemeinen wird das frisch zugeführte Synthesegas so geteilt, dass über die jeweiligen Teilströme gleiche Mengen an Synthesegas zugeführt werden.

Die pro Zeiteinheit zugeführte gesamte Synthesegasmenge sowie die über die zuvor erwähnten Teilströme zugeführte Synthesegasmenge hängt von den jeweiligen Reaktordimensionen ab und kann durch einfache Versuche ermittelt werden.

In einer besonderen Ausgestaltung des erfindungsgemäßen Verfahrens wird das aus der Extraktionskolonne zurückgeführte Synthesegas über den Reaktorboden eingespeist, während frisches Synthesegas auf den Mittelteil des Hydroformylierungsreaktors gegeben wird. Der mittlere Bereich des Hydroformylierungsreaktors, auf den Synthesegas gegeben wird, erstreckt sich in einer Zone oberhalb und unterhalb vom Mittelpunkt des Hydroformylierungsreaktors, die bis zu 30 %, vorzugsweise bis zu 20 % der gesamten Reaktorlänge, beträgt.

Überraschenderweise bewirkt die Zufuhr von Synthesegas auf den Mittelteil des Hydroformylierungsreaktors sowohl eine deutliche Steigerung des Umsatzes an olefinisch ungesättigter Verbindung zu Aldehyden als auch eine Selektivitätssteigerung zu den geradkettigen Aldehyden.

Nach dem erfindungsgemäßen Verfahren setzt man dem nach dem Gas/Flüssigabscheider erhaltenen flüssigen, homogen Stoffstrom vor dem Eintritt in die Extraktionskolonne Synthesegas zu. Der Synthesegaszusatz führt bereits in der Zuleitung zu der Extraktionskolonne zu einer weiteren Umsetzung der olefinisch ungesättigten Verbindung zu Aldehyden unter Freisetzung von Reaktionswärme, so dass die Temperatur des flüssigen Stoffstromes vor dem Eintritt in die Extraktionskolonne nochmals erhöht werden kann, ohne dass eine externe Energiezufuhr erforderlich ist. Dabei wird Synthesegas in einer solchen Menge zugeführt, die im allgemeinen 5-40%, vorzugsweise 10-20%, der in den Hydroformylierungsreaktor eingespeisten Synthesegasmenge beträgt.

Das erfindungsgemäße Verfahren kann auf olefinisch ungesättigte Verbindungen beliebiger Struktur angewandt werden. Dementsprechend sind als Ausgangsmaterial geeignet sowohl Olefine mit innenständiger als auch mit endständiger Doppelbindung und ebenso geradkettige wie verzweigte Olefine. Überdies können die Olefine auch noch funktionelle Gruppen enthalten, insbesondere solche, die im Verlauf der Reaktion nicht verändert werden.

Auch mehrfach olefinisch ungesättigte Verbindungen kommen als Einsatzstoffe in Betracht, wie z.B. 1,3-Butadien oder 1,3 Pentadien. Besonders bewährt hat sich das erfindungsgemäße Verfahren zur Hydroformylierung olefinisch ungesättigter Kohlenwasserstoffe mit 3 bis 12 Kohlenstoffatomen im Molekül, vorzugsweise Propylen und die isomeren Butene, die in der Technik als Raffinat-II, einem Gemisch aus Buten-1 und Buten-2, oder als Raffinat-III, mit dem man ein Buten-1 abgereichertes Raffinat-II bezeichnet, verfügbar sind. Aber auch ein Octen-2 und/oder Octen-3 enthaltendes C₈-Olefingemisch, läßt sich nach dem erfindungsgemäßen Verfahren als Ausgangsverbindung einsetzen. Auch die technisch verfügbaren Olefingemische, wie Dimersol ® oder Octol ® oder Trimerpropylen lassen sich in dem erfindungsgemäßen Verfahren umsetzen.

Rhodium gelangt entweder als Metall oder als Verbindung zum Einsatz. In metallischer Form verwendet man es entweder als feinverteilte Partikel oder in dünner Schicht auf einem Träger, wie Aktivkohle, Calciumcarbonat, Aluminiumsilikat, Tonerde, niedergeschlagen. Als Rhodiumverbindungen eignen sich Salze aliphatischer Mono- und Polycarbonsäuren, wie Rhodium-2-ethyl-hexanoat, Rhodiumacetat, Rhodiumoxalat, Rhodiumpropionat oder Rhodiummalonat. Weiterhin können Rhodiumsalze anorganischer Wasserstoff- oder Sauerstoffsäuren, wie Rhodiumnitrat oder Rhodiumsulfat, die verschiedenen Rhodiumoxide oder auch Rhodiumcarbonylverbindungen wie Rh₃(CO)₁₂ oder Rh₆(CO)₁₆ oder Komplexverbindungen des Rhodiums, z.B. Cyclooctadienylrhodiumverbindungen oder Rhodiumacetylacetonat, eingesetzt werden. Rhodiumhalogenverbindungen kommen wegen des korrosiven Verhaltens der Halogenidionen weniger in Betracht. Bevorzugt werden Rhodiumoxid und insbesondere Rhodiumnitrat, Rhodiumacetat und Rhodium-2-ethylhexanoat.

In den beigefügten Zeichnungen werden beispielhaft mögliche Ausführungsformen des erfindungsgemäßen Verfahrens schematisch dargestellt.

Nach dem in Bild 1 skizzierten Verfahrensablauf werden dem mit Flüssigkeit vollständig gefüllten Hydroformylierungsreaktor 1 am Boden über die Leitung 2 Olefin zugeführt. Der über die Leitung 3 zugeführte frische Synthesegasstrom wird in dem Splitter 4 in den über die Leitung 5 auf den Boden des Hydroformylierungsreaktors 1 geführten Teilstrom und in den über die Leitung 6 auf den Boden der Extraktionskolonne 12 geführten Teilstrom geteilt. Am Kopf des Hydroformylierungsreaktors 1 wird der flüssige, Katalysator haltige Produktstrom über die Leitung 7 entnommen und auf einen Gas/Flüssigabscheider 8 gegeben. Das dabei anfallende Abgas wird über die Leitung 9 abgeführt, es enthält überwiegend Kohlenmonoxid, Wasserstoff und in geringen Anteilen nicht umgesetzte olefinisch ungesättigte Verbindung und Reaktionsprodukt. Der flüssige Produktstrom wird über die Leitung 10 mit Hilfe einer Pumpe 11 auf die Extraktionskolonne 12 gegeben. Über die Leitung 6 wird Synthesegas am Boden der Extraktionskolonne 12 eingeführt und es strömt dem flüssigen Produktstrom entgegen, wobei es zu einer weiteren Umsetzung der im flüssigen Produktstrom gelösten Restmenge an olefinisch ungesättigter Verbindung zu Aldehyden kommt. In der Extraktionskolonne 12 wird ebenfalls Wärme von dem heißen, flüssigen Produktstrom auf das den am Kopf der Extraktionskolonne 12 verlassende Synthesegas über tragen, das über die Leitung 13 zusammen mit Restspuren an olefinisch ungesättigter Verbindung wieder in den Hydroformylierungsreaktor 1 zurückgeführt wird. Der von der olefinisch ungesättigten Verbindung befreite Produktstrom, enthaltend Rohaldehyd, Lösungsmittel und Katalysator wird über die Leitung 14 auf den Verdampfer 15 gegeben, in dem der Rohaldehyd verdampft und über die Leitung 16 zur weiteren Aufarbeitung, die in an sich bekannter Weise erfolgt, abgeführt wird. Die im Verdampfer anfallende hochsiedende Lösung, die die hochsiedenden Aldehydkondensationsprodukte und gegebenenfalls andere, zugesetzte hochsiedende Lösungsmittel und den gelösten Rhodiumkatalysator enthält, wird über die Leitung 17 mit Hilfe der Pumpe 18 wieder in den Hydroformylierungsreakor 1 zurückgeführt.

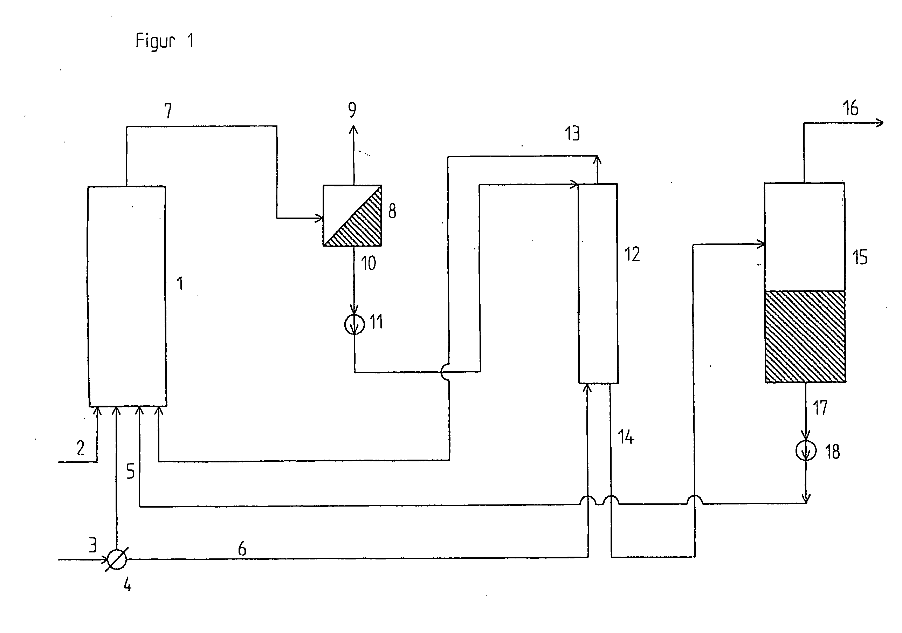

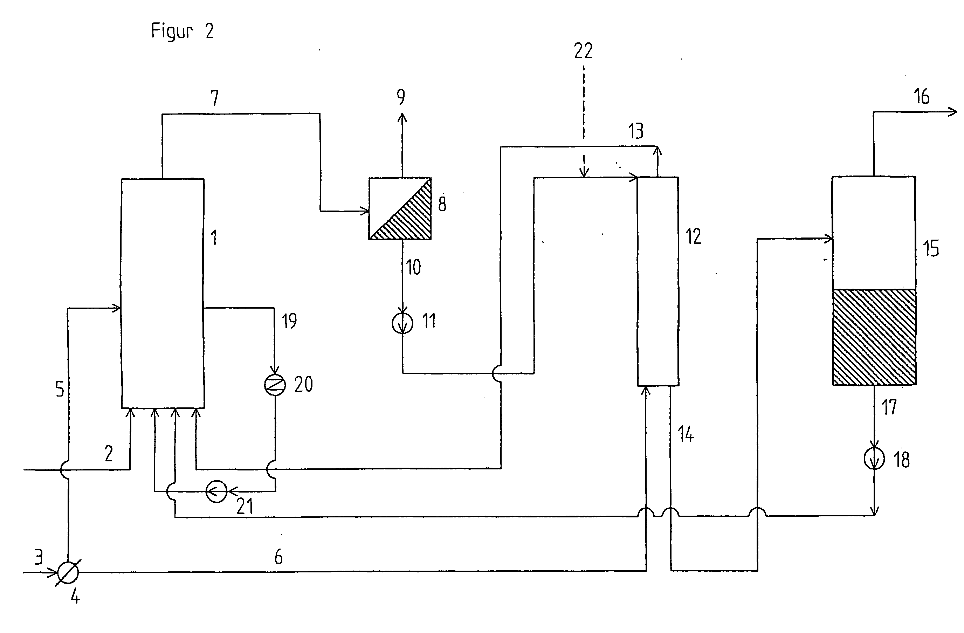

Die in Figur 2 dargestellte Verfahrensvariante unterscheidet sich vom Prozeßablauf gemäß Figur 1 dadurch, dass der Hydroformylierungsreaktor 1 als Schlaufenreaktor betrieben wird. Dazu wird ein flüssiger Teilstrom über die Leitung 19 abgeführt, in dem Wärmetauscher 20 abgekühlt und mit Hilfe der Pumpe 21 wieder dem Reaktor zugeführt, um einen Flüssigkeitskreislauf aufrechtzuerhalten. Bei der in Figur 2 dargestellten Verfahrensvariante wird frisches Synthesegas über die Leitung 3 herangeführt und der in dem Splitter 4 anfallende Teilstrom Synthesegas über die Leitung 5 nunmehr auf den Mittelteil des Hydroformylierungsreaktors 1 gegeben. Nach dem erfindungsgemäßen Verfahren wird über die Leitung 22, die in Figur 2 gestrichelt dargestellt ist, vor der Aufgabestelle auf die Extraktionskolonne 12 zusätzlich Synthesegas in die Leitung 10 eingespeist. Die mit den Ziffern 2, 6 bis 9, 11, 13 bis 18 bezeichneten Anlagenteile entsprechen denen der Figur 1.

Die Erfindung wird in den nachfolgenden Beispielen näher erläutert, jedoch nicht auf die beschriebenen Ausführungsformen beschränkt.

### Beispiel 1 (Vergleich)

In der gemäß Figur 2 dargestellten Ausführung wurde unter folgenden Reaktionsbedingungen die Hydroformylierung von Propylen durchgeführt.

| | |
|---|---|
| Reaktorvolumen: | 14 Liter |
| Druck: | 2,1 MPa |
| Temperatur: | 120°C |
| Synthesegaszufuhr (gesamt): | 4 Nm³/h* |
| | |
| Propylenzufuhr: | 5,0 kg/h |
| Verweilzeit des flüssigen Reaktorinhalts: | 0,7 h |
| | |
| Rhodiumkonzentration: | 600 ppm |
| Triphenylphosphin-Konzentration, bez. auf die gesamte Reaktionslösung | 30 % |

| | |
|---|---|
| (*) Die Angabe Nm³/h bedeutet Normkubikmeter pro Stunde, d.h. 1 Kubikmeter bei einer Temperatur von 273 K und einem Druck von 1013 mbar. | |

In den mit Katalysatorlösung gefüllten Hydroformylierungsreaktor 1 wurde über die Leitung 2 Propylen vom Boden her eingeführt. Man leitete frisches Synthesegas über die Leitung 3 heran und führte es durch den Splitter 4, wobei der Splitter so eingestellt war, dass die gesamte über die Leitung 3 herangeführte Synthesegasmenge auf die Leitung 6 gegeben wurde. Das gesamte zugeführte Synthesegas strömte zunächst durch die Extraktionskolonne 12 und anschließend über die Leitung 13 auf den Boden des Hydroformylierungsreaktors 1. Über den Flüssigkeitskreislauf 19 wurde der Hydroformylierungsreaktor 1 nach dem Prinzip des Schlaufenreaktors betrieben, wobei die Reaktionswärme über den Wärmetauscher 20 abgeführt wurde. Der Propylenumsatz im Hydroformylierungsreaktor betrug 85 %. Der flüssige Reaktoraustrag wurde über die Leitung 7 in den Gas/Flüssigabscheider 8 eingeleitet, in dem Abgas über die Leitung 9 abgeführt wurde. Die Temperatur des anfallenden flüssigen Stoffstroms, der etwa 5 Gew.-% Propylen, bezogen auf die homogene, flüssige Lösung, enthielt, lag bei etwa 115°C. Über die Leitung 10 strömte diese Lösung mit Hilfe der Pumpe 11 auf den 1. Boden der Extraktionskolonne, gerechnet vom Kolonnenkopf. Dem flüssigen Stoffstrom wurde über die Leitung 6 im Gegenstrom Synthesegas entgegengeführt. Die Reaktionstemperatur konnte in einer Zone von 20 %, bezogen auf die Länge der Extraktionskolonne und gerechnet vom Kolonnenkopf, aufrechterhalten werden. Das Kopfprodukt der Extraktionskolonne, enthaltend überwiegend Synthesegas und Propylen, wurde über die Leitung 13 auf den Boden des Hydroformylierungsreaktors 1 zurückgeführt. Der flüssige Austrag aus der Extraktionskolonne 12 wies eine Temperatur von 85°C auf und enthielt noch 0,3 Gew.-% Restpropylen, bezogen auf die gesamte Flüssigkeitsmenge. Diesen flüssigen Stoffstrom gab man über die Leitung 14 auf den Verdampfer 15, der bei einer Temperatur von 145°C und bei Normaldruckruck betrieben wurde, wobei Rohaldehyd als Kopfprodukt über die Leitung 16 anfiel und Katalysatorlösung über die Leitung 17 mit Hilfe der Pumpe 18 in den Hydroformylierungsreaktor 1 zurückgeschleust wurde. Der über die Leitung 16 abgeführte Rohaldehyd wurde in bekannter Weise zunächst kondensiert, wobei das Abgas, enthaltend Propylenrestmengen, Inerte, zum Beispiel Propan, und andere flüchtige Bestandteile abgeführt wurde, während der flüssige Rohaldehyd weiter aufgearbeitet wurde (nicht in Figur 2 gezeigt).

### Beispiel 2

Es wurde analog Beispiel 1 gearbeitet, mit der einzigen Ausnahme, dass über die Leitung 22 zusätzlich Synthesegas in die Leitung 10 eingespeist wurde. Die über Leitung 22 zugeführte Synthesegasmenge lag zwischen 10-20%, bezogen auf den Synthesegaseinsatz in den Hydroformylierungsreaktor. Der Synthesegaszusatz bewirkte einen weiteren Propylenumsatz in der Leitung 10 und führte zu einem Temperaturanstieg auf 142°C und zu einer Abnahme des Restpropylengehaltes auf 4 Gew.-%, bezogen auf die gesamte Lösung. Im Vergleich zu der Ausführung gemäß Beispiel 1 wies der in die Extraktionskolonne 12 über die Leitung 10 eingeführte homogene, flüssige Stoffstrom somit eine höhere Temperatur und einen geringeren Propylenrestgehalt auf. Ebenfalls, im Vergleich zu Beispiel 1, besaß der die Extraktionskolonne 12 über die Leitung 14 verlassende flüssige Stoffstrom eine auf 115°C angestiegene Temperatur. Propylen konnte in ihm nicht mehr nachgewiesen werden.

### Beispiel 3

Beispiel 3 wurde wie das Beispiel 2 durchgeführt, mit der einzigen Ausnahme, dass in dem Splitter 4 das zugeführte Synthesegas hälftig geteilt und ein Synthesegasteilstrom über die Leitung 5 auf den Mittelteil des Hydroformylierungsreaktors 1 gegeben wurde. Im Vergleich zu Beispiel 1 konnte der Propylenumsatz um 6,5 % auf 90,5 % gesteigert werden, während die übrigen Ergebnisse den Ergebnissen aus Beispiel 2 entsprachen.

## Patentansprüche

1. Verfahren zur Umsetzung olefinisch ungesättigter Verbindungen mit Synthesegas in Gegenwart einer Rhodium und organische Phosphor(III)-Verbindungen enthaltenden Katalysatorlösung in einem Hydroformylierungsreaktor, bei dem der homogene, flüssige Rohaldehyd und Katalysator enthaltende Reaktoraustrag auf einen Gas/Flüssigabscheider gegeben wird und der erhaltene homogene, flüssige Stoffstrom in einer mit Synthesegas im Gegenstrom betriebenen Extraktionskolonne behandelt wird und der flüssige Austrag aus der Extraktionskolonne in einen Verdampfer geleitet wird, in dem Rohaldehyd und Katalysatorlösung voneinander getrennt werden, **dadurch gekennzeichnet, dass** dem nach dem Gas/Flüssigabscheider anfallenden homogenen, flüssigen Stoffstrom vor dem Eintritt in die Extraktionskolonne Synthesegas zugesetzt wird und der homogene, flüssige Stoffstrom bei einer Temperatur von 110 bis 150°C auf den oberen Teil der Extraktionskolonne gegeben wird.

2. Verfahren nach Anspruch 1, **dadurch gekennzeichnet, dass** Synthesegas an einer oder an mehreren Einspeisestellen dem Hydroformylierungsreaktor zugeführt wird.

3. Verfahren nach einem der Ansprüche 1 oder 2, **dadurch gekennzeichnet, dass** sich das dem Hydroformylierungsreaktor zugeführte Synthesegas aus frischem Synthesegas und dem über die Extraktionskolonne zurückgeführten Synthesegas zusammensetzt.

4. Verfahren nach einem oder mehreren der Ansprüche 1 bis 3, **dadurch gekennzeichnet, dass** frisches Synthesegas auf den Mittelteil des Hydroformylierungsreaktors gegeben wird und das über die Extraktionskolonne zurückgeführte Synthesegas über den unteren Teil des Hydroformylierungsreaktors zugeführt wird.

5. Verfahren nach einem oder mehreren der Ansprüche 1 bis 4, **dadurch gekennzeichnet, dass** der nach dem Gas/Flüssigabscheider anfallende, homogene, flüssige Stoffstrom auf den ersten Boden der Extraktionskolonne, gerechnet vom Kolonnenkopf, gegeben wird.

6. Verfahren nach einem oder mehreren der Ansprüche 1 bis 5, **dadurch gekennzeichnet, dass** die Extraktionskolonne eine Bodenkolonne, eine Packungskolonne oder eine Füllkörperkolonne ist.

7. Verfahren nach einem oder mehreren der Ansprüche 1 bis 6, **dadurch gekennzeichnet, dass** die Extraktionskolonne 10 bis 40, vorzugsweise 15 bis 25 Trennstufen besitzt.

8. Verfahren nach einem oder mehreren der Anprüche 1 bis 7, **dadurch gekennzeichnet, dass** die Konzentration an organischen Phosphor(III)-Verbindungen in der homogenen Reaktionslösung von 15 bis 60 Gew.-%, bezogen auf die homogene Reaktionslösung, beträgt.

9. Verfahren nach Anpruch 8, **dadurch gekennzeichnet, dass** die Konzentration an organischen Phosphor(III)-Verbindungen in der homogenen Reaktionslösung von 25 bis 50 Gew.-%, bezogen auf die homogene Reaktionslösung, beträgt.

10. Verfahren nach einem oder mehreren der Ansprüche 1 bis 9, **dadurch gekennzeichnet, dass** die Rhodiumkonzentration in der homogenen Reaktionslösung von 1 bis 1000 ppm, bezogen auf die homogene Reaktionslösung beträgt.

11. Verfahren nach Anspruch 10, **dadurch gekennzeichnet, dass** die Rhodiumkonzentration in der homogenen Reaktionslösung von 10 bis 800 ppm, bezogen auf die homogene Reaktionslösung beträgt.

## Claims

1. A process for reacting olefinically unsaturated compounds with synthesis gas in the presence of a catalyst solution comprising rhodium and organic phosphorus(III) compounds in a hydroformylation reactor, in which the homogeneous, liquid reactor output comprising crude aldehyde and catalyst is passed to a gas/liquid separator and the homogeneous, liquid stream obtained is treated in an extraction column operated in countercurrent with synthesis gas and the liquid output from the extraction column is fed into a vaporizer in which crude aldehyde and catalyst solution are separated from one another, wherein synthesis gas is added to the homogeneous, liquid stream obtained from the gas/liquid separator before this stream enters the extraction column, and the homogeneous, liquid stream is introduced at a temperature of from 110 to 150°C, into the upper part of the extraction column.

2. The process as claimed in claim 1, wherein synthesis gas is fed into the hydroformylation reactor at one or more feed points.

3. The process as claimed in claim 1 or 2, wherein the synthesis gas fed to the hydroformylation reactor is composed of fresh synthesis gas and the synthesis gas recirculated via the extraction column.

4. The process as claimed in one or more of claims 1 to 3, wherein fresh synthesis gas is introduced into the middle part of the hydroformylation reactor and the synthesis gas which is recirculated via the extraction column is fed in via the lower part of the hydroformylation reactor.

5. The process as claimed in one or more of claims 1 to 4, wherein the homogeneous liquid stream obtained from the gas/liquid separator is introduced on the first tray of the extraction column, counted from the top of the column.

6. The process as claimed in one or more of claims 1 to 5, wherein the extraction column is a tray column, a column containing ordered packing or a column containing random packing elements.

7. The process as claimed in one or more of claims 1 to 6, wherein the extraction column has from 10 to 40, preferably from 15 to 25, theoretical plates.

8. The process as claimed in one or more of claims 1 to 7, wherein the concentration of organic phosphorus(III) compounds in the homogeneous reaction solution is from 15 to 60% by weight, based on the homogeneous reaction solution.

9. The process as claimed in claim 8, wherein the concentration of organic phosphorus(III) compounds in the homogeneous reaction solution is from 25 to 50% by weight, based on the homogeneous reaction solution.

10. The process as claimed in one or more of claims 1 to 9, wherein the rhodium concentration of the homogeneous reaction solution is from 1 to 1000 ppm, based on the homogeneous reaction solution.

11. The process as claimed in claim 10, wherein the rhodium concentration of the homogeneous reaction solution is from 10 to 800 ppm, based on the homogeneous reaction solution.

## Revendications

1. Procédé de conversion de composés oléfiniquement insaturés à l'aide de gaz de synthèse en présence d'une solution de catalyseur contenant du rhodium et des composés organiques du phosphore (III) dans un réacteur d'hydroformylation, lors duquel la décharge de réacteur contenant l'aldéhyde brut liquide homogène et le catalyseur est transférée sur un séparateur gaz/liquides, le courant de susbtances liquide homogène obtenu est traité dans une colonne d'extraction fonctionnant à contre-courant à l'aide de gaz de synthèse et la décharge liquide provenant de la colonne d'extraction est conduite dans un évaporateur, dans lequel l'aldéhyde brut et la solution de catalyseur sont séparés l'un de l'autre, **caractérisé en ce que** l'on ajoute au courant de susbtances liquide homogène se formant après le séparateur gaz/liquides, avant l'entrée dans la colonne d'extraction, du gaz de synthèse et **en ce que** l'on introduit le courant de susbtances liquide homogène à une température de 110 à 150°C sur la partie supérieure de la colonne d'extraction.

2. Procédé selon la revendication 1, **caractérisé en ce que** le gaz de synthèse est alimenté au réacteur d'hydroformylation à un ou plusieurs emplacements d'injection.

3. Procédé selon l'une quelconque des revendications 1 ou 2, **caractérisé en ce que** le gaz de synthèse alimenté au réacteur d'hydroformylation se compose de gaz de synthèse frais et du gaz de synthèse alimenté par l'intermédiaire de la colonne d'extraction.

4. Procédé selon l'une ou plusieurs des revendications 1 à 3, **caractérisé en ce que** l'on fournit du gaz de synthèse frais à la partie médiane du réacteur d'hydroformylation et **en ce que** l'on alimente le gaz de synthèse reconduit par l'intermédiaire de la colonne d'extraction par le biais de la partie inférieure du réacteur d'hydroformylation.

5. Procédé selon l'une ou plusieurs des revendications 1 à 4, **caractérisé en ce que** le courant de susbtances liquide homogène se formant après le séparateur gaz/liquides est introduit sur le premier plateau de la colonne d'extraction, par calcul à partir de la tête de la colonne.

6. Procédé selon l'une ou plusieurs des revendications 1 à 5, **caractérisé en ce que** la colonne d'extraction est une colonne à plateaux, une colonne à garniture ou une colonne à corps de remplissage.

7. Procédé selon l'une ou plusieurs des revendications 1 à 6, **caractérisé en ce que** la colonne d'extraction possède de 10 à 40, de préférence, de 15 à 25 étages de séparation.

8. Procédé selon l'une ou plusieurs des revendications 1 à 7, **caractérisé en ce que** la concentration en composés organiques du phosphore (III) dans la solution de réaction homogène est de 15 à 60 % en poids, par rapport à la solution de réaction homogène.

9. Procédé selon la revendication 8, **caractérisé en ce que** la concentration en composés organiques du phosphore (III) dans la solution de réaction homogène est de 25 à 50 % en poids, par rapport à la solution de réaction homogène.

10. Procédé selon l'une ou plusieurs des revendications 1 à 9, **caractérisé en ce que** la concentration en rhodium dans la solution de réaction homogène est de 1 à 1000 ppm, par rapport à la solution de réaction homogène.

11. Procédé selon la revendication 10, **caractérisé en ce que** la concentration en rhodium dans la solution de réaction homogène est de 10 à 800 ppm, par rapport à la solution de réaction homogène.
